# EUROPEAN PATENT APPLICATION

(11) **EP 4 088 773 A2**
(43) Date of publication of application: **16.11.2022**
(21) Application number: 20912079.9
(22) Date of filing: 24.12.2020
(51) Int. Cl.: A61M 37/00, A61K 9/00, A61M 19/00

(54) **MICRONEEDLE APPLICATOR**

(30) Priority: 09.01.2020 KR 20200003119
(71) Applicant: Industry-Academic Cooperation Foundation, Yonsei University, Seoul 03722 (KR); Juvic Inc., Seoul 08389 (KR)
(72) Inventor: JUNG, Hyung Il, Seoul 02838 (KR); YANG, Hui Suk, Seoul 06514 (KR); KIM, Hyeon Jun, Seoul 08280 (KR)
(74) Representative: Kobiako von Gamm, Iouri
(86) International application number: PCT/KR2020/019113
(87) International publication number: WO 2021/141304

(57) **Abstract**

A microneedle applicator is provided. A microneedle applicator according to an embodiment of the present invention comprises: a microneedle array; an operation member disposed on the upper portion of the microneedle array and operating such that a user applies an external force so as to insert a microneedle into skin; a housing for accommodating the microneedle array and the operation member; a first guide member disposed in at least one position on a lateral portion of the operation member or on a lateral portion of an interworking member of the operation member; and a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

## Description

### CROSS-REFERENCE TO RELATED APPLICATION

This application claims priority to and the benefit of Korean Patent Application No. 10-2020-0003119, filed on January 9, 2020, the disclosure of which is incorporated herein by reference in its entirety.

### [Technical Field]

The present invention relates to a microneedle applicator, and more particularly to a microneedle applicator for uniformly inserting microneedles into the skin to ensure the reliability of drug delivery.

### [Background Art]

Biodegradable microneedles, which have been recently researched and developed, are usually inserted into the skin in two types. That is, a patch form and an applicator are used. Herein, in the case of the patch form, problems have occurred such as skin irritation caused by patch attachment and detachment, a decrease in the insertion rate of microneedles caused by human hair pushing a patch out of the skin where human hair is present and interfering with the adhesion of the patch and the skin, difficulty in attaching a patch where there are flexion and movement of the skin such as wrinkles or joints and the like.

Moreover, since the microneedle patch is inserted into the skin by the pressing force of the user's palm, it does not guarantee the insertion reliability of microneedles depending on the user or the attachment site.

In particular, when the hardness of microneedles is low, the degree of insertion of the microneedles is not uniform depending on the position of the microneedle patch, and thus, the efficiency of drug delivery is lowered.

### [Disclosure]

### [Technical Problem]

In order to solve the problems of prior art as described above, an exemplary embodiment of the present invention is to provide a microneedle applicator which is capable of quickly and effectively delivering a drug contained in the microneedle to the skin.

In addition, an exemplary embodiment of the present invention is to provide a microneedle applicator which is capable of ensuring stable insertion reliability of the microneedles into the skin even when the hardness is low.

However, the problems to be solved by the present invention are not limited to the problems mentioned above, and other problems that are not mentioned will be clearly understood by those skilled in the art from the following description.

### [Technical Solution]

According to an aspect of the present invention to solve the aforementioned problems, provided is a microneedle applicator, including a microneedle array; an operation member disposed on the upper portion of the microneedle array and operating such that a user applies an external force so as to insert a microneedle into skin; a housing for accommodating the microneedle array and the operation member; a first guide member disposed in at least one position on a lateral portion of the operation member or on a lateral portion of an interworking member of the operation member; and a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

In an exemplary embodiment, the microneedle may have a hardness of 0.5N or less.

In an exemplary embodiment, the microneedle array may include a drug, and the drug may include a drug for local anesthesia selected from the group consisting of cocaine, procaine, chloroprocaine, tetracaine, dibucaine, lidocaine, mepivacaine, benzocaine, bupivacaine and a combination thereof.

In an exemplary embodiment, the first guide member may be provided in a straight-line shape in a vertical direction from a lateral portion of the operation member.

In an exemplary embodiment, the second guide member may have a different width depending on a position on the horizontal cross-section of the housing.

In an exemplary embodiment, the microneedle applicator may further include an adhesive layer provided on the lower portion of the housing and attached to the skin, wherein the adhesive layer may have an opening in the center and be provided with a peeling part such that one side protrudes to the outside.

In an exemplary embodiment, the microneedle applicator may further include a locking member provided to support the lower end of the operation member, wherein the operation member may press the microneedle array with a force of passing through the locking member by the external force.

In an exemplary embodiment, the locking member may be provided to protrude from the upper end of the inner surface of the housing toward the center.

In an exemplary embodiment, the locking member may be vertically provided in a rod shape from the inside of the housing, wherein the operation member may be provided with a groove portion at a position corresponding to the locking member, and is provided with a support to protrude into the groove portion, and wherein the support may be broken by the external force.

In an exemplary embodiment, the locking member may be a groove portion provided on the upper end of the inner surface of the housing, and the operation member may be provided with a protrusion that is inserted into the groove portion along the outer periphery.

In an exemplary embodiment, the microneedle applicator may further include a fluid layer provided between the operation member and the microneedle array, wherein the fluid layer may support the lower end of the operation member, and wherein the operation member may press the microneedle array with a force of destroying the fluid layer by the external force.

In an exemplary embodiment, the fluid layer may include air, a liquid and a drug.

In an exemplary embodiment, the microneedle applicator may further include a rubber band provided between the operation member and the microneedle array, wherein the operation member may fix the rubber band such that the rubber band has elasticity on the upper side of the microneedle array, and wherein when the rubber band is released by the external force, the operation member may press the microneedle array by an elastic restoring force of the rubber band.

In an exemplary embodiment, the microneedle applicator may further include a leaf spring provided between the operation member and the microneedle array, wherein the operation member may press the microneedle array with a force of elastically deforming the leaf spring by the external force.

In an exemplary embodiment, the microneedle applicator may further include a support for supporting the microneedle array between the leaf spring and the microneedle array

In an exemplary embodiment, the microneedle array may be a microneedle patch.

According to another aspect of the present invention, provided is a microneedle applicator, including a microneedle array; an operation member disposed on the upper portion of the microneedle array and operating such that a user applies an external force so as to insert a microneedle into skin; a housing for accommodating the microneedle array and the operation member; and a support member provided between the microneedle array and the operation member to support the operation member with a constant supporting force and release the supporting force by the external force such that the operation member joins the microneedle array.

In an exemplary embodiment, the microneedle applicator may further include a first guide member disposed in at least one position on a lateral portion of the operation member; and a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

In an exemplary embodiment, the support member may be a locking member provided to protrude from the upper end of the inner surface of the housing toward the center, wherein the locking member may support the lower end of the operation member, and wherein the operation member may press the microneedle array with a force of releasing a supporting force by the locking member by the external force.

In an exemplary embodiment, the support member may be a fluid layer, and the operation member may press the microneedle array with a force of releasing a supporting force by the fluid layer by the external force.

In an exemplary embodiment, the skin in contact with the housing may expand to the outside by the support force.

According to still another aspect of the present invention, provided is a microneedle applicator, including a microneedle array; an operation member disposed on the upper portion of the microneedle array and operating such that a user applies an external force so as to insert a microneedle into skin; a housing for accommodating the microneedle array and the operation member; and an elastic member provided between the microneedle array and the operation member to press the microneedle array with an elastic force caused by the external force.

In an exemplary embodiment, the microneedle applicator may further include a first guide member disposed in at least one position on a lateral portion of the operation member or on a lateral portion of an interworking member of the operation member; and a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

In an exemplary embodiment, the elastic member may be a rubber band, wherein the operation member may fix the rubber band such that the rubber band has elasticity on the upper side of the microneedle array, and wherein when the rubber band is released by the external force, the operation member may press the microneedle array by an elastic restoring force of the rubber band.

In an exemplary embodiment, the elastic member may be a leaf spring, and when the leaf spring is pressed by the external force, the operation member may press the microneedle array by an elastic deformation force of the leaf spring.

According to still another aspect of the present invention, provided is a microneedle array applicator, including a microneedle array; an operation member disposed on the upper portion of the microneedle array and operating such that a user applies an external force so as to insert a microneedle into skin; and a housing for accommodating the microneedle array and the operation member, wherein the operation member and the opening of the housing have the same shape, and the operation member is disposed to be staggered at a predetermined angle such that the operation member is supported by an opening edge of the housing, and wherein the support of the operation member is released according to the rotation of the operation member by the external force such that the operation member slides along the opening of the housing so as to prevent the microneedle array from rotating when the microneedle array uniformly vertically descends.

### [Advantageous Effects]

The microneedle applicator according to an exemplary embodiment of the present invention inserts a microneedle into the skin by using an external force applied by the user such that the drug contained in the microneedle array can be delivered quickly and effectively, thereby minimizing the user's discomfort.

In addition, since the present invention provides a vertical sliding guide, an external force can be uniformly applied to the entire microneedle array such that it is possible to ensure insertion reliability even when the hardness of the microneedle is low, thereby improving drug delivery efficiency.

In addition, since the present invention provides an external force with a relatively simple structure by providing a support member between the operation member to which an external force is applied by the user and the microneedle array, and providing an external force to the microneedle array through release of the support force of the support member, it is possible to reduce the manufacturing cost and thus improve the price competitiveness.

In addition, since the present invention provides an elastic member at the upper portion of the microneedle array and provides an external force to the microneedle array through the elastic force of the elastic member such that the force applied by the user is required relatively small, it is possible to improve the convenience of use.

In addition, since the present invention includes a drug for local anesthesia in the microneedle, it is possible to anesthetize quickly and painlessly when the drug for local anesthesia is administered, and thus, it is possible to perform the injection painlessly.

### [Description of Drawings]

FIG. 1 is an exploded perspective view of the microneedle applicator according to a first example of the present invention.
FIG. 2 is a horizontal cross-sectional view showing another example of the guide member of FIG. 1.
FIG. 3 is a view for describing the operation of the microneedle applicator according to the first example of the present invention.
FIG. 4 is an exploded perspective view of the microneedle applicator according to a second example of the present invention.
FIG. 5 is a view for describing the operation of the microneedle applicator according to the second example of the present invention.
FIG. 6 is an exploded perspective view of the microneedle applicator according to a third example of the present invention.
FIG. 7 is a view for describing the operation of the microneedle applicator according to the third example of the present invention.
FIG. 8 is an exploded perspective view of the microneedle applicator according to a fourth example of the present invention.
FIG. 9 is a view for describing the operation of the microneedle applicator according to the fourth example of the present invention.
FIG. 10 is a view showing the microneedle applicator according to a fifth example of the present invention.

### [Modes of the Invention]

Hereinafter, exemplary embodiments of the present invention will be described in detail with reference to the accompanying drawings such that those of ordinary skill in the art to which the present invention pertains may easily practice the present invention. The present invention may be implemented in various different forms, and is not limited to the exemplary embodiments described herein. In the drawings, parts irrelevant to the description are omitted in order to clearly describe the present invention, and the same reference numerals are assigned to the same or similar components throughout the specification.

The exemplary embodiments of the present invention are provided to describe the present invention more completely to those of ordinary skill in the art, and the exemplary embodiments described below may be modified in various other forms, and the scope of the present invention is not limited to the following exemplary embodiments. Rather, these exemplary embodiments are provided to more fully and completely describe the present invention, and to fully convey the spirit of the present invention to those skilled in the art.

In the drawings, variations from the illustrated shapes may be expected as a result of, for example, manufacturing techniques and/or tolerances. Thus, the exemplary embodiments of the present invention should not be construed as being limited to the particular shapes of regions illustrated herein but may include deviations in shapes that result, for example, from manufacturing processes.

The microneedle applicator according to the present invention is for effectively inserting the microneedle array into the skin and includes four examples. In this case, the microneedle applicator according to the present invention provides an additional external force to increase the reliability of the microneedle insertion into the skin. As an example, the microneedle array may be a microneedle patch. In addition, the microneedle array may include a drug. In this case, the drug may be a drug for various uses as a biodegradable material.

Herein, the first example and the second example provide an external force as a force corresponding to the supporting force by a support member, the third example and the fourth example provide an external force as an elastic force by an elastic member, and the fifth example provides an external force as a force corresponding to the supporting force while omitting a separate support member.

Accordingly, the microneedle applicator according to the present invention may deliver a drug contained in the microneedle array quickly and effectively because the microneedle can be stably inserted into the skin. Therefore, it is possible to minimize the user's discomfort according to the use of the microneedle array.

Meanwhile, since the microneedle including a drug for local anesthesia has a relatively low hardness, the microneedle cannot be inserted normally in some parts of the microneedle array compared to other parts. That is, the microneedle may be nonuniformly inserted in the microneedle array.

To this end, as a result of repeated intensive research and experiments, the inventors of the present invention have completed the present invention by discovering that the microneedle must be uniformly leveled throughout the microneedle array while the microneedle is inserted into the skin.

In the present specification, the drug for local anesthesia is not particularly limited as an anesthetic drug for anesthetizing a body part. For example, the drug for local anesthesia may be selected from the group consisting of cocaine, procaine, chloroprocaine, tetracaine, dibucaine, lidocaine, mepivacaine, benzocaine, bupivacaine and a combination thereof.

As a result, the microneedle applicator according to the present invention may quickly and painlessly anesthetize even when the drug itself for local anesthesia is administered, and it may be performed painlessly when the hypodermic needle or catheter is inserted by local anesthesia.

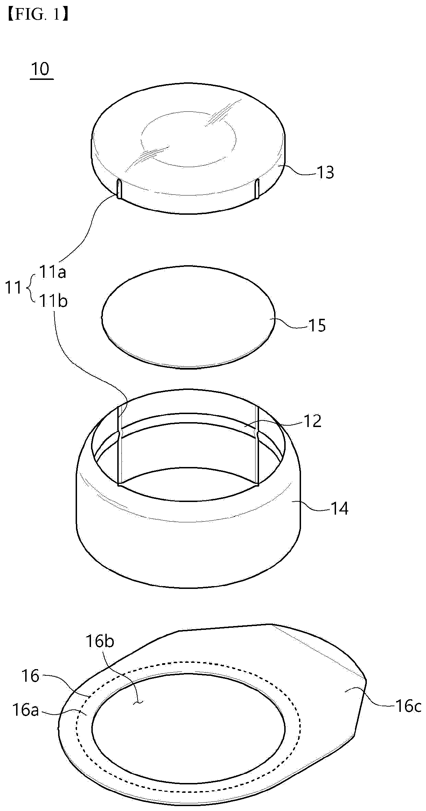

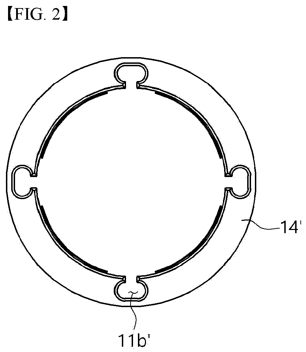

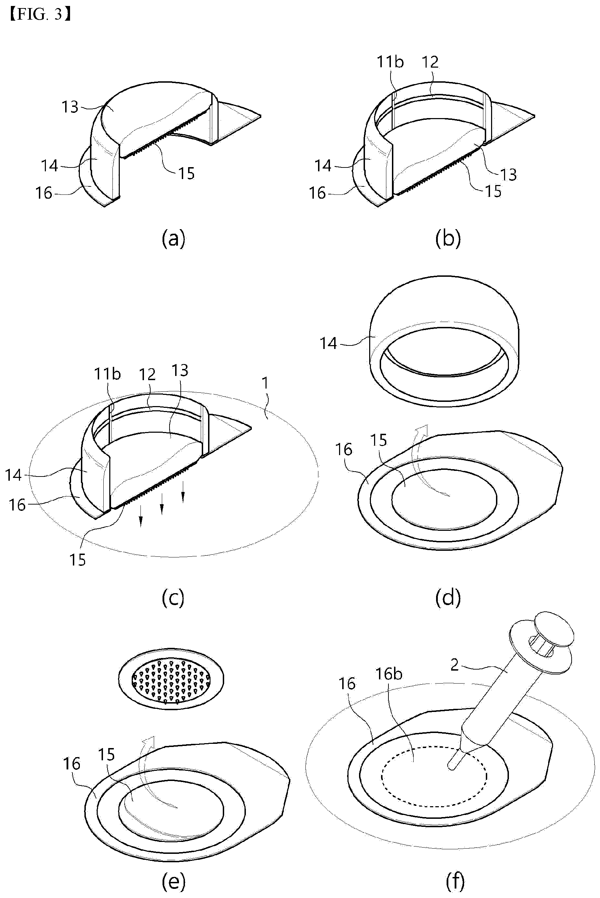

FIG. 1 is an exploded perspective view of the microneedle applicator according to a first example of the present invention, FIG. 2 is a horizontal cross-sectional view showing another example of the guide member of FIG. 1, and FIG. 3 is a view for describing the operation of the microneedle applicator according to the first example of the present invention.

Referring to FIG. 1, the microneedle applicator 10 according to the first example of the present invention includes a guide member 11, a locking member 12, an operation member 13, a housing 14 and a microneedle array 15.

The microneedle applicator 10 according to the first example is for providing an external force by a support member, and a locking member 12 is provided as a support member.

The guide member 11 includes a first guide member 11a and a second guide member 11b.

The first guide member 11a is provided in at least one position on a lateral portion of the operation member 13. For example, the first guide member 11a may be provided to protrude in a protrusion shape. Conversely, the first guide member 11a may be provided concavely in the form of a groove. In this case, the first guide member 11a may be provided in at least three positions of the operation member 13 in order to maintain the overall balance of the operation member 13.

In addition, the first guide member 11a may be provided in a straight-line shape in a vertical direction from the side of the operation member 13. For example, the first guide member 11a may be provided in a "1" shape. That is, the first guide member 11a may be provided in a straight line from the upper surface to the lower surface of the operation member 13. However, the first guide member 11a is not limited thereto and may have various shapes.

The second guide member 11b is provided on the inner surface of the housing 14 at a position corresponding to the first guide member 11a. For example, the second guide member 11b may be provided concavely in the form of a groove. Conversely, the second guide member 11b may be provided to protrude in a protrusion shape. Herein, the second guide member 11b may be provided in a vertical direction from the inner surface of the housing 14. In addition, the second guide member 11b has a shape corresponding to the first guide member 11a, and the first guide member 11a is inserted thereinto.

In this case, the second guide member 11b allows the first guide member 11a to slide such that the microneedle array 15 uniformly descends through the operation member 13 by an external force applied by the user.

As a result, the microneedle applicator 10 according to the first example of the present invention may uniformly apply an external force with respect to the entire microneedle array 15. Therefore, even when the hardness of the microneedle is low, since the microneedle can be uniformly inserted into the skin as a whole, the insertion reliability of the microneedle may be guaranteed, thereby improving the drug delivery efficiency.

In the present specification, the detailed description of the guide member 11 will be described with reference to the first example, but certainly, it may also be applied to the second to fourth examples.

Referring to FIG. 2, the second guide member 11b' may have a different width depending on a position on the horizontal cross-section of the housing 14'. That is, the second guide member 11b may have a greater width from the outside than the inside of the housing 14'. In this case, the first guide member 11a may have a shape corresponding to the second guide member 11b'.

For example, the second guide member 11b' and the first guide member 11a may have a "T" shape, but are not limited thereto and may have various shapes.

As described above, by increasing the lengths of the surfaces of the second guide member 11b' and the first guide member 11a facing each other, the possibility that the first guide member 11a may be separated from the second guide member 11b' is minimized, and thus, the vertical descending characteristic of the operation member 13 may be further improved.

The locking member 12 supports the lower end of the operation member 13. Herein, the locking member 12 may support the operation member 13 with a constant supporting force. In this case, the locking member 12 may be provided with a size sufficient to allow the operation member 13 to pass through the locking member 12 by an external force.

For example, as illustrated in FIG. 1, the locking member 12 is provided to protrude from the upper end of the inner surface of the housing 14 toward the center. That is, the locking member 12 may be provided in the shape of a locking jaw. In this case, the locking member 12 may be formed entirely or in part along the inner circumferential surface of the housing 14.

As another example, the locking member 12 may be vertically provided in a rod shape from the inside of the housing 14. In this case, the operation member 13 is provided with a groove portion at a position corresponding to the locking member 12, and the support may be provided to protrude to the inside of the groove portion. Herein, the support may be broken by an external force.

As another example, the locking member 12 may be a groove portion provided at the upper end of the inner surface of the housing 14. In this case, the operation member 13 may be provided with a protrusion that is inserted into the groove portion along the outer periphery.

The operation member 13 is provided on the upper end of the housing 14 and is provided at an upper portion the microneedle array 15. The operation member 13 operates such that an external force is applied by the user to insert the microneedle into the skin. In this case, the operation member 13 may release the supporting force of the locking member 12 by an external force such that the operation member 13 presses the microneedle array 15. In this way, the operation member 13 may be operated by the user. In addition, the operation member 13 may have a disk shape.

In this case, the operation member 13 may have a concave shape in the center of the upper surface. That is, a concave portion may be provided in the center of the upper surface of the operation member 13 in order to guide the user's finger to be located in the center of the operation member 13.

As a result, the external force applied by the user may uniformly act on the entire operation member 13. Accordingly, it is possible to further ensure uniform descending of the microneedle array 15.

In addition, the operation member 13 may press the microneedle array 15 with a force passing through the locking member 12 by an external force. In other words, the operation member 13 may press the microneedle array 15 with a force of releasing the supporting force by the locking member 12.

The housing 14 accommodates the operation member 13 and the microneedle array 15. In this case, the operation member 13 may be exposed to the outside from the upper end of the housing 14 so as to be operated by the user. In addition, the housing 14 has an overall cylindrical shape, and a hollow portion for accommodating the operation member 13 and the microneedle array 15 may be provided at the center thereof. That is, the housing 14 may be opened at both the upper side and the lower side.

In addition, the housing 14 may be provided at a constant height such that the operation member 13 vertically descends while preventing rotation or horizontal torsion to provide a sufficient force to insert the microneedles of the microneedle array 15 into the skin. Herein, the housing 14 may be provided with an inner surface accommodating the operation member 13 and the microneedle array 15 as a vertical surface.

The microneedle array 15 is provided with a plurality of microneedles including a drug. Herein, the microneedle may be made of a biodegradable material. In addition, the drug may be a drug for local anesthesia as described above, but may include drugs for other uses. In this case, the microneedle may have a hardness of 0.5N or less. In addition, the microneedle may have a shape suitable for insertion into the skin. As an example, the microneedle may have a conical shape, but is not limited thereto.

In addition, the microneedle array 15 may be disposed at a lower portion of the operation member 13. In this case, the microneedle array 15 may be attached to the operation member 13 through an adhesive layer.

The microneedle applicator 10 may further include an adhesive layer 16.

The adhesive layer 16 may be provided at a lower portion of the housing 14. In addition, an adhesive may be applied to the lower surface of the adhesive layer 16. As a result, the adhesive layer 16 may be attached to the skin. In this case, the adhesive layer 16 may be covered by a protective layer.

In addition, the adhesive layer 16 may be provided with an adhesive portion 16a along the periphery of the opening 16b on the upper surface. Herein, the adhesive portion 16a may have the same shape as the open lower surface of the housing 14. For example, the adhesive portion 16a may have a circular band shape.

In addition, the adhesive layer 16 may have an opening 16b in the center. The opening 16b allows the microneedle array 15 or the microneedles of the microneedle array 15 to be exposed to the skin. Herein, the opening 16b may have a circular shape, but is not particularly limited thereto.

In addition, the adhesive layer 16 is provided with a peeling part 16c such that one side protrudes to the outside. In this case, the peeling part 16c may not have an adhesive applied thereto so as to easily separate the adhesive layer 16 from the skin. In addition, the peeling part 16c may record a memo such as a patient's condition.

Hereinafter, the operation of the microneedle applicator 10 will be described with reference to FIG. 3.

Referring to FIG. 3, as shown in (a), the protective layer on the lower surface of the adhesive layer 16 is removed and the microneedle array 15 is prepared to be applied to the skin. As shown in (b), when the operation member 13 is pushed by the user, the operation member 13 passes through the locking member 12 and vertically descends to the lower surface of the housing 14 so as to prevent rotation or horizontal distortion.

In this case, by the supporting force of the locking member 12, the side wall of the housing 14 may expand the skin in contact therewith to the outside. As a result, by inserting the microneedle into the expanded skin, it is possible to improve the insertion efficiency and ensure the insertion safety at the same time.

As shown in (c), the microneedle array 15 is in contact with the skin 1 according to the descending of the operation member 13. In this case, the microneedle is inserted into the skin 1 by the force of the operation member 13 passing through the locking member 12. As shown in (d), the microneedle applicator 10 is removed. In this case, when the housing 14 is removed, only the adhesive layer 16 and the microneedle array 15 remain on the skin 1.

As shown in (e), when the microneedle array 15 is removed, the skin 1 is exposed through the opening 16b while the adhesive layer 16 is attached to the skin 1 as shown in (f). In this case, in order to inject a different drug from the microneedle array 15 or collect body fluid, a hypodermic injection needle 2 is inserted into the exposed skin.

As such, the microneedle applicator 10 according to the first example of the present invention may provide an external force to the microneedle array 15 through the release of the supporting force of the locking member 12. Therefore, since the microneedle applicator 10 according to the first example of the present invention may provide an external force with a relatively simple structure, it is possible to reduce the manufacturing cost, and thus improve the price competitiveness.

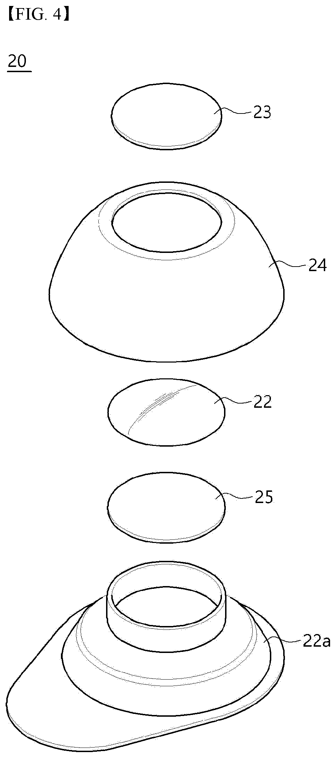

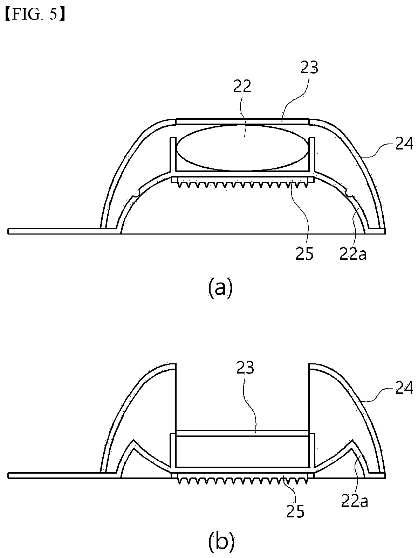

FIG. 4 is an exploded perspective view of the microneedle applicator according to a second example of the present invention, and FIG. 5 is a view for describing the operation of the microneedle applicator according to the second example of the present invention.

Referring to FIG. 4, the microneedle applicator 20 according to the second example of the present invention includes a fluid layer 22, an operation member 23, a housing 24 and a microneedle array 25.

The microneedle applicator 20 according to the second example is for providing an external force by a support member, and a fluid layer 22 is provided as a support member.

The fluid layer 22 is provided between the operation member 23 and the microneedle array 25. Herein, the fluid layer 22 may be a capsule in which any one of air, a liquid and a drug is injected. In this case, the capsule may be formed in an oval shape with a convex center.

In addition, the fluid layer 22 supports the lower end of the operation member 23. Herein, the fluid layer 22 may support the operation member 23 with a constant supporting force. In this case, the fluid layer 22 may be injected with air at a pressure sufficient to maintain the support force.

In addition, the fluid layer 22 may be provided at an upper portion of a movable member 22a. Herein, the fluid layer 22 may be disposed at the upper portion of the movable member 22a, and the microneedle array 25 may be disposed at the lower portion of the movable member 22a. In this case, the movable member 22a supports the fluid layer 22 and at the same time is pressed by the operation member 23 according to the destruction of the fluid layer 22, thereby vertically descending the microneedle array 25 to prevent rotation or horizontal distortion.

In addition, the movable member 22a may include a space for accommodating the fluid layer 22. That is, the fluid layer 22 may be seated in the space of the movable member 22a. Herein, the space may be formed by a vertically formed sidewall.

In addition, the movable member 22a may have a central portion convex from the lower portion of the housing 24. Herein, the movable member 22a may be a leaf spring having an elastic force. In this case, the movable member 22a may be supported by a force greater than the destructive force of the fluid layer 22.

In addition, the movable member 22a may be elastically deformed downward by an external force. That is, the movable member 22a may be provided with an elastic force on a curved inclined surface connecting the lower surface and the convex upper surface. Herein, the movable member 22a may be elastically deformed downward to provide a sufficient force to insert the microneedles of the microneedle array 25 into the skin at a constant height.

In addition, the movable member 22a may be provided such that one side protrudes to the outside. A note such as a patient's condition may be recorded on this protrusion.

The operation member 23 is provided on the upper end of the housing 24 and is provided at an upper portion of the microneedle array 25. The operation member 23 operates such that an external force is applied by the user to insert the microneedle into the skin. In this case, the operation member 23 may release the supporting force of the fluid layer 22 by an external force such that the operation member 23 presses the microneedle array 25. In this way, the operation member 23 may be operated by the user. In addition, the operation member 23 may have a disk shape.

In this case, the operation member 23 may have a concave shape in the center of the upper surface. That is, a concave portion may be provided in the center of the upper surface of the operation member 23 in order to guide the user's finger to be located at the center of the operation member 23.

As a result, the external force applied by the user may uniformly act on the entire operation member 23. Accordingly, it is possible to further ensure uniform descending of the microneedle array 25.

In addition, the operation member 23 may press the microneedle array 25 with a force of destroying the fluid layer 22 by an external force. In other words, the operation member 23 may press the microneedle array 25 with a force of releasing the supporting force by the fluid layer 22. In this case, the operation member 23 may press the microneedle array 25 through the movable member 22a.

The housing 24 accommodates the operation member 23 and the microneedle array 25. In this case, the operation member 23 may be exposed to the outside from the upper end of the housing 24 so as to be operated by the user. Herein, the movable member 22a may be disposed to support the lower end of the housing 24. That is, the housing 24 may be disposed above the movable member 22a. As a result, the housing 24 is not in direct contact with the skin.

In addition, the housing 24 has an overall cylindrical shape, and a hollow portion for accommodating the operation member 23 and the microneedle array 25 may be provided at the center thereof. Herein, the housing 24 has a first hollow portion for accommodating the fluid layer 22 and the operation member 23 at the upper portion, and a second hollow portion for accommodating the movable member 22a and the microneedle array 25 at the lower portion. In this case, the width of the first hollow portion may be smaller than the width of the second hollow portion. In addition, the housing 24 may be open from both the upper and lower sides.

In addition, the housing 24 may be provided at a constant height such that the operation member 23 vertically descends while preventing rotation or horizontal distortion so as to provide a sufficient force to insert the microneedles of the microneedle array 25 into the skin. Herein, the housing 24 may be provided with an inner surface accommodating the operation member 23 and the microneedle array 25 as a vertical surface.

The microneedle array 25 is provided with a plurality of microneedles including a drug. Herein, the microneedle may be made of a biodegradable material. In addition, the drug may be a drug for local anesthesia as described above. In this case, the microneedle may have a hardness of 0.5N or less. In addition, the microneedle may have a shape suitable for insertion into the skin. As an example, the microneedle may have a conical shape, but is not limited thereto.

The microneedle array 25 may be disposed on the lower surface of the central convex portion of the movable member 22a. That is, the microneedle array 25 may be disposed on the lower surface of a position corresponding to the fluid layer 22 accommodated in the movable member 22a. In this case, the microneedle array 25 may be attached to the movable member 22a through an adhesive layer.

Meanwhile, although not illustrated in the drawings, the microneedle applicator 20 according to the second example may be provided with the guide member 11 and the adhesive layer 16 of the first example.

Herein, the first guide member may be provided to protrude in at least one position on the side of the operation member 23. In addition, the second guide member is provided on the inner surface of the housing 24 at a position corresponding to the first guide member.

In addition, the second guide member has a shape corresponding to the first guide member, and the first guide member is inserted thereinto. In this case, the second guide member allows the first guide member to slide such that the microneedle array 25 uniformly descends through the operation member 23 and the movable member 22a by an external force applied by the user.

Herein, it is certain that the first guide member and the second guide member may be provided in shapes opposite to each other such that the first guide member has a groove shape and the second guide member has a protrusion shape.

In addition, the adhesive layer may be provided at a lower portion of the movable member 22a. The adhesive layer may be attached to the skin by applying an adhesive on the lower surface.

Hereinafter, the operation of the microneedle applicator 20 will be described with reference to FIG. 5.

Referring to FIG. 5, as shown in (a), while the fluid layer 22 supports the operating member 23 in the microneedle applicator 20, the movable member 22a supports the fluid layer 22 and the operation member 23 by a central convex portion.

In this case, by the supporting force of the fluid layer 22, the side wall of the housing 24 may expand the skin in contact therewith to the outside. As a result, by inserting the microneedle into the expanded skin, it is possible to improve the insertion efficiency and ensure the insertion safety at the same time.

As shown in (b), when the operation member 23 is pushed by the user, the operation member 23 destroys the fluid layer 22 and vertically descends to the lower surface of the housing 14 to prevent rotation or horizontal distortion. In this case, since the supporting force of the movable member 22a is greater than the pressure of the fluid layer 22, the fluid layer 22 may be destroyed between the operation member 23 and the movable member 22a.

Accordingly, by the operation member 23, the center of the movable member 22a vertically descends by the elastic deformation of the inclined surface such that the microneedle array 25 protrudes to the outside of the housing 24. In this case, the microneedle array 25 is in contact with the skin, and the microneedle is inserted into the skin by the force of the operation member 23 destroying the fluid layer 22.

As such, the microneedle applicator 20 according to the second example of the present invention may provide an external force to the microneedle array 25 through the release of the supporting force of the fluid layer 22. Therefore, since the microneedle applicator 20 according to the second example of the present invention may provide an external force with a relatively simple structure, it is possible to reduce the manufacturing cost and thus improve the price competitiveness.

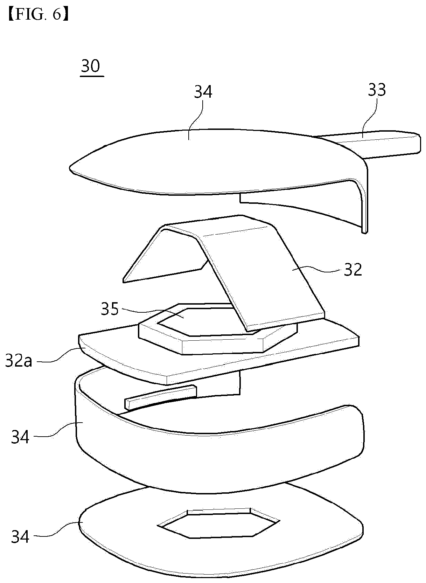

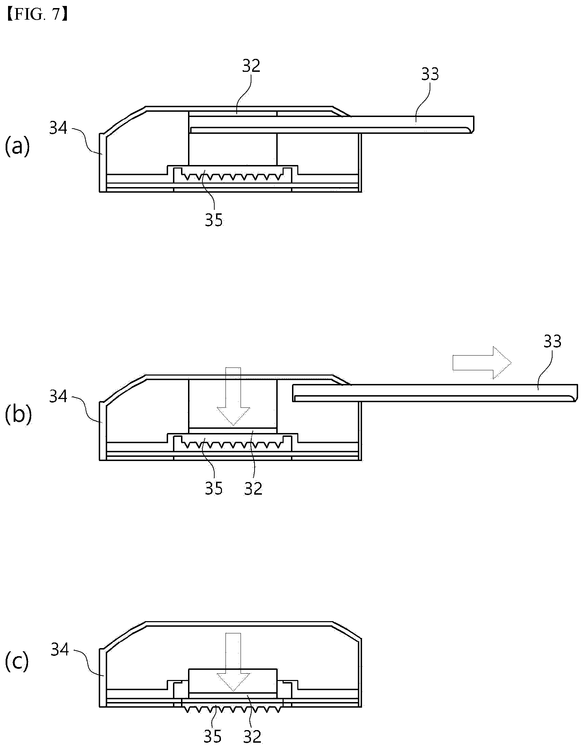

FIG. 6 is an exploded perspective view of the microneedle applicator according to a third example of the present invention, and FIG. 7 is a view for describing the operation of the microneedle applicator according to the third example of the present invention.

Referring to FIG. 6, the microneedle applicator 30 according to the third example of the present invention includes a rubber band 32, an operation member 33, a housing 34 and a microneedle array 35.

The microneedle applicator 30 according to the third example is for providing an external force by an elastic member, and a rubber band 32 is provided as an elastic member.

The rubber band 32 is provided between the operation member 33 and the microneedle array 35. Herein, the rubber band 32 may be formed in a linear shape having a constant width.

The rubber band 32 may have a central portion fixed by the operation member 33. In addition, the rubber band 32 may have the other side fixed to an interlocking member 32a. In this case, the rubber band 32 may be elastically deformed by the distance between the operation member 33 and the interlocking member 32a to generate an elastic force. Herein, the rubber band 32 may be diagonally disposed between the operation member 33 and the interlocking member 32a on one side and between the operation member 33 and the interlocking member 32a on the other side.

The interlocking member 32a may support the microneedle array 35 under the microneedle array 35. In addition, the interlocking member 32a may be disposed on the upper side of the microneedle array 35. In this case, the microneedle array 35 may be attached to the interlocking member 32a. Herein, the interlocking member 32a may be provided with a through hole in the center such that the microneedle array 35 is exposed downward.

In addition, the interlocking member 32a has a function of transmitting the elastic force by the rubber band 32 to the microneedle array 35. In this case, the interlocking member 32a may vertically descend the microneedle array 35 according to the release of the rubber band 32.

In addition, the interlocking member 32a may be formed in a plate shape. Herein, the interlocking member 32a may have a greater strength than the elastic force of the rubber band 32. Therefore, the shape is not deformed even by the elastic deformation force of the rubber band 32.

The operation member 33 is provided on the upper end of the housing 34 and is provided on the upper side of the microneedle array 35. The operation member 33 operates such that an external force is applied by the user to insert the microneedles into the skin. In this case, the operation member 33 provides the elastic restoring force of the rubber band 32 so as to press the microneedle array 35 with an elastic force by an external force.

In addition, the operation member 33 fixes the central portion of the rubber band 32 such that the rubber band 32 has elasticity on the upper side of the microneedle array 35. Herein, the operation member 33 may be horizontally slidably disposed. That is, the operation member 33 may slide in a direction perpendicular to the rubber band 32. In this way, the operation member 33 may be operated by the user.

In this case, when the operation member 33 horizontally moves to the outside of the housing 34 by the user's external force, the rubber band 32 may be released. Therefore, it is possible to press the microneedle array 35 through the interlocking member 32a by the elastic restoring force of the rubber band 32.

Herein, the operation member 33 has been described as an example of horizontal movement to the outside of the housing 34 in the form of a horizontal bar, but is not particularly limited thereto, and it may have a structure capable of providing an elastic force to the rubber band 32.

For example, the operation member 33 may be provided in the form of a vertical bar to vertically move upwards of the housing 34. In this case, the rubber band 32 is elastically deformed in the vertical direction according to the vertical movement of the operation member 33 while being disposed on the lower side of the housing 34. Afterwards, as the external force is removed from the operation member 33, the microneedle array 35 may be pressed by the elastic restoring force of the rubber band 32. Herein, the rubber band 32 may be supported by the operation member 33 and the interlocking member 32a.

As another example, the operation member 33 may be provided in a form having a rotational force to rotate on the upper side of the housing 34. In this case, the rubber band 32 is elastically deformed as it rotates within the housing 34. Afterwards, as the external force is removed from the operation member 33, the microneedle array 35 may be pressed by the elastic restoring force of the rubber band 32. Herein, the rubber band 32 may be supported by the operation member 33 and the interlocking member 32a.

The housing 34 accommodates the operation member 33 and the microneedle array 35. In this case, the operation member 33 may be exposed to the outside from the upper end of the housing 34 so as to be operated by the user. Herein, the housing 34 is illustrated as an upper end, a side wall and a lower end, but it is substantially provided integrally. Accordingly, the housing 34 has an overall cylindrical shape.

In addition, the housing 34 may be provided with a hollow portion for accommodating the rubber band 32, the interlocking member 32a and the microneedle array 35 in the center. That is, the housing 34 may be opened at both the upper side and the lower side.

In addition, the housing 34 may be provided at a constant height such that the interlocking member 32a vertically descends to provide a sufficient force for the microneedles of the microneedle array 35 to be inserted into the skin. Herein, the housing 34 may be provided with an inner surface accommodating the interlocking member 32a and the microneedle array 35 as a vertical surface.

The microneedle array 35 is provided with a plurality of microneedles including a drug. Herein, the microneedle may be made of a biodegradable material. In addition, the drug may be a drug for local anesthesia as described above. In this case, the microneedle may have a hardness of 0.5N or less. In addition, the microneedle may have a shape suitable for insertion into the skin. As an example, the microneedle may have a conical shape, but is not limited thereto.

In addition, the microneedle array 35 may be disposed in the central portion of the interlocking member 32a. In this case, the microneedle array 35 may be attached to the upper and lower sides of the interlocking member 32a through an adhesive layer.

Meanwhile, although not illustrated in the drawings, the microneedle applicator 30 according to the third example may be provided with the guide member 11 and the adhesive layer 16 of the first example.

Herein, the first guide member may be provided to protrude in at least one position of a lateral portion of the interlocking member 32a. In addition, the second guide member is provided on the inner surface of the housing 34 at a position corresponding to the first guide member.

In addition, the second guide member has a shape corresponding to the first guide member, and the first guide member is inserted thereinto. In this case, the second guide member allows the first guide member to slide such that the microneedle array 35 uniformly descends through the operation member 33 and the interlocking member 32a by an external force applied by the user.

Herein, it is certain that the first guide member and the second guide member may be provided in shapes opposite to each other such that the first guide member has a groove shape and the second guide member has a protrusion shape.

In addition, the adhesive layer may be provided at a lower portion of the housing 34. The adhesive layer may be attached to the skin by applying an adhesive on the lower surface.

Hereinafter, the operation of the microneedle applicator 30 will be described with reference to FIG. 7.

Referring to FIG. 7, as shown in (a), the microneedle applicator 30 has both ends of the rubber band 32 fixed to the interlocking member 32a, and the central portion is fitted to the operation member 33. In this case, the rubber band 32 is elastically deformed by the operation member 33 and the interlocking member 32a. Herein, the microneedle array 35 is disposed near the lower surface of the housing 34 integrally with the interlocking member 32a. Optionally, the microneedle array 35 may be disposed near the upper surface of the housing 34 integrally with the interlocking member 32a.

As shown in (b), when the operation member 33 slides outward by the user, the central portion of the rubber band 32 that has been fixed by the operation member 33 is released and the rubber band 32 applies a force downward by the elastic restoring force.

As shown in (c), the elastic restoring force of the rubber band 32 is transmitted to the interlocking member 32a such that the microneedle array 35 protrudes to the outside of the lower surface of the housing 34. Accordingly, the microneedle array 35 is in contact with the skin, and the microneedle is inserted into the skin by the elastic restoring force of the rubber band 32.

As such, the microneedle applicator 30 according to the third example of the present invention may provide an external force to the microneedle array 35 through the elastic restoring force of the rubber band 32. Therefore, the microneedle applicator 30 according to the third example of the present invention may provide a sufficient external force for the user to insert the microneedle into the skin with only a small force, thereby improving the convenience of use.

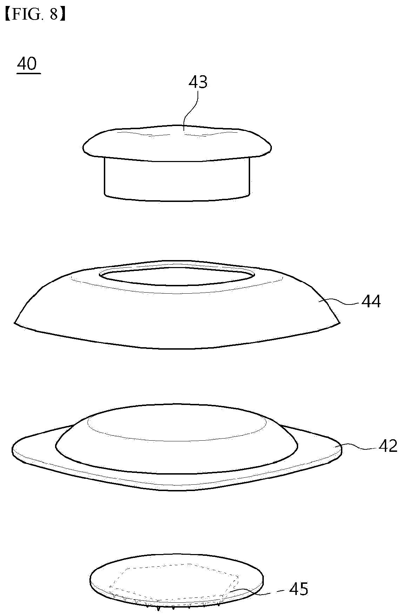

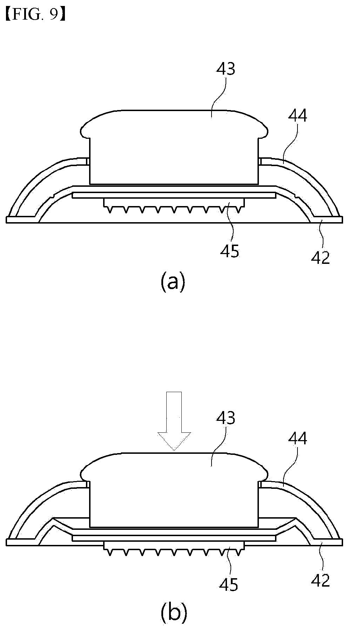

FIG. 8 is an exploded perspective view of the microneedle applicator according to a fourth example of the present invention, and FIG. 9 is a view for describing the operation of the microneedle applicator according to the fourth example of the present invention.

Referring to FIG. 8, the microneedle applicator 40 according to the fourth example of the present invention includes a leaf spring 42, an operation member 43, a housing 44 and a microneedle array 45.

The microneedle applicator 40 according to the fourth example is for providing an external force by an elastic member, and the leaf spring 42 is provided as an elastic member.

The leaf spring 42 is provided between the operation member 43 and the microneedle array 45. Herein, the leaf spring 42 may have a convex central portion. In this case, the central portion of the leaf spring 42 may be elastically deformed downward by an external force. That is, the leaf spring 42 may provide an elastic force on the curved inclined surface connecting the lower surface and the convex upper surface. Herein, the leaf spring 42 may be elastically deformed downward to provide a sufficient force to insert the microneedles of the microneedle array 45 into the skin. In this case, the leaf spring 42 may be elastically deformed by the height of the convex portion to generate an elastic force.

The leaf spring 42 supports the lower end of the operation member 43. Herein, the leaf spring 42 may support the operation member 43 with a supporting force corresponding to the elastic force of the curved inclined surface.

The operation member 43 is provided on the upper end of the housing 44 and is provided on the microneedle array 45. The operation member 43 operates such that an external force is applied by the user to insert the microneedle into the skin. In this case, the operation member 43 presses the leaf spring 42 so as to press the microneedle array 45 with an elastic force by an external force to provide an elastic deformation force. In this way, the operation member 43 may be operated by the user. In addition, the operation member 43 may have a disk shape.

In this case, the operation member 43 may have a shape in which the central portion of the upper surface is concave. That is, a concave portion may be provided in the center of the upper surface of the operation member 43 in order to guide the user's finger to be located at the center of the operation member 43.

As a result, the external force applied by the user may uniformly act on the entire operation member 43. Accordingly, it is possible to further ensure uniform descending of the microneedle array 45.

In addition, the operation member 43 may press the microneedle array 45 with a force that elastically deforms the leaf spring 42 by an external force. In other words, when the operation member 43 presses the leaf spring 42 by an external force, the microneedle array 45 may be pressed by the elastic deformation force of the leaf spring 42.

The housing 44 accommodates the leaf spring 42, the operation member 43 and the microneedle array 45. In this case, the operation member 43 may be exposed to the outside from the upper end of the housing 44 so as to be operated by the user. Herein, the leaf spring 42 may be disposed to support the lower end of the housing 44. That is, the housing 44 may be disposed at an upper portion of the leaf spring 42. As a result, the housing 44 is not in direct contact with the skin.

The housing 44 has an overall cylindrical shape, and a hollow portion for accommodating the leaf spring 42, the operation member 43 and the microneedle array 45 may be provided at the center thereof. Herein, the housing 44 may be provided with a first hollow portion for accommodating the operation member 43 at an upper portion, and a second hollow portion for accommodating the leaf spring 42 and the microneedle array 45 at a lower portion. In this case, the width of the first hollow part may be smaller than the width of the second hollow part. In addition, the housing 44 may be open at both the upper and lower sides.

The housing 44 may be provided at a constant height such that the operation member 43 vertically descends to provide a sufficient force to insert the microneedles of the microneedle array 45 into the skin. Herein, the housing 44 may be provided with an inner surface accommodating the operation member 43 as a vertical surface.

The microneedle array 45 is provided with a plurality of microneedles including a drug. Herein, the microneedle may be made of a biodegradable material. In addition, the drug may be a drug for local anesthesia as described above. In this case, the microneedle may have a hardness of 0.5N or less. In addition, the microneedle may have a shape suitable for insertion into the skin. As an example, the microneedle may have a conical shape, but is not limited thereto.

In addition, the microneedle array 45 may be disposed on the lower surface of the convex portion of the center of the leaf spring 42. In this case, the microneedle array 45 may be attached to the leaf spring 42 through an adhesive layer.

Alternatively, the microneedle array 45 may be provided on a separate support. In this case, the support may be provided between the leaf spring 42 and the microneedle array 45. That is, the microneedle array 45 may be provided separately from the leaf spring 42.

Meanwhile, although not illustrated in the drawings, the microneedle applicator 40 according to the fourth example may be provided with the guide member 11 and the adhesive layer 16 of the first example.

Herein, the first guide member may be provided to protrude in at least one position on a lateral portion of the operation member 43. In addition, the second guide member is provided on the inner surface of the housing 44 at a position corresponding to the first guide member.

In addition, the second guide member has a shape corresponding to the first guide member, and the first guide member is inserted thereinto. In this case, the second guide member allows the first guide member to slide such that the microneedle array 45 uniformly descends through the operation member 43 and the leaf spring 42 by an external force applied by the user.

Herein, it is certain that the first guide member and the second guide member may be provided in shapes opposite to each other such that the first guide member has a groove shape and the second guide member has a protrusion shape.

In addition, the adhesive layer may be provided at a lower portion of the leaf spring 42. The adhesive layer may be attached to the skin by applying an adhesive on the lower surface.

Hereinafter, the operation of the microneedle applicator 40 will be described with reference to FIG. 9.

Referring to FIG. 9, as shown in (a), in the microneedle applicator 40, the operation member 43 protrudes to the upper portion of the housing 44, and the leaf spring 42 supports the operation member 43 by the central convex portion.

As shown in (b), when the operation member 43 is pushed by the user, the operation member 43 presses the central portion of the leaf spring 42. In this case, the inclined surface of the leaf spring 42 is elastically deformed, and the central portion thereof vertically descends downward such that the microneedle array 45 protrudes to the outside of the housing 44. Accordingly, the microneedle array 45 is in contact with the skin, and the microneedle is inserted into the skin by the elastic deformation force of the leaf spring 42.

As such, the microneedle applicator 40 according to the fourth example of the present invention may provide an external force to the microneedle array 45 through the elastic deformation force of the leaf spring 42. Therefore, the microneedle applicator 40 according to the fourth example of the present invention may improve the convenience of use, because the user can provide a sufficient external force to insert the microneedle into the skin with only a small force.

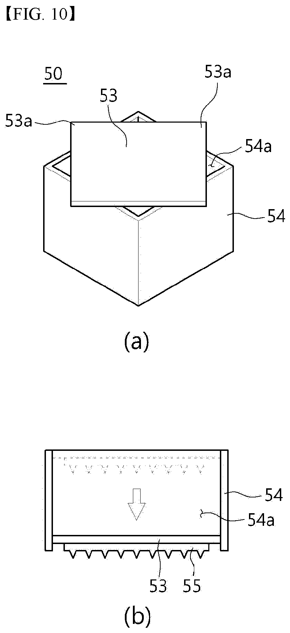

FIG. 10 is a view showing the microneedle applicator according to a fifth example of the present invention.

Referring to FIG. 10, as shown in (a), the microneedle applicator 50 according to the fifth example of the present invention includes an operation member 53, a housing 54 and a microneedle array 55.

The microneedle applicator 50 according to the fifth example is provided with a guide member and a locking member integrally.

The operation member 53 is provided on the upper end of the housing 54, and is provided on the microneedle array 55. Herein, the operation member 53 may have the same shape as the opening 54a of the housing 54. That is, the outer periphery of the operation member 53 may have the same shape as the inner periphery of the opening 54a of the housing 54. In addition, the operation members 53 may be staggered at a predetermined angle so as to be supported by the corners of the opening 54a of the housing 54. In this case, the portion 53a located outside the opening 54a of the housing 54 and the edge of the housing 54 may function as a locking member. For example, the operation member 53 may have a polygonal shape, and preferably, a rectangular shape. As another example, the operation member 53 may include a curved outer periphery thereof. Preferably, the operation member may be circular.

In this case, the operation member 53 may have a shape in which the central portion of the upper surface is concave. That is, a concave portion may be provided at the center of the upper surface of the operation member 53 in order to guide the user's finger to be located at the center of the operation member 53.

As a result, the external force applied by the user may uniformly act on the entire operation member 53. Accordingly, it is possible to further ensure uniform descending of the microneedle array 55.

In addition, the operation member 53 may be rotatably coupled to the housing 54. That is, the operation member 53 may be rotated by an external force. Herein, when the operation member 53 and the opening 54a of the housing 54 coincide with each other, the supporting force by the corners of the portion 53a of the operation member 53 and the housing 54 may be released. Accordingly, the operation member 53 may press the microneedle array 55 by an external force.

The housing 54 accommodates the operation member 53 and the microneedle array 55. For example, the housing may have an opening 54a having the same shape as that of the operation member 53. Herein, the opening 54a may be provided along the central portion of the housing 54.

The microneedle array 55 is provided with a plurality of microneedles including a drug. Herein, the microneedle may be made of a biodegradable material. In addition, the drug may be a drug for local anesthesia as described above. In this case, the microneedle may have a hardness of 0.5N or less. In addition, the microneedle may have a shape suitable for insertion into the skin. As an example, the microneedle may have a conical shape, but is not limited thereto.

In addition, the microneedle array 55 may be disposed at a lower portion of the operation member 53. In this case, the microneedle array 55 may be attached to the operation member 53 through an adhesive layer.

Meanwhile, although not illustrated in the drawings, the microneedle applicator 50 according to the fifth example may be provided with the guide member 11 and the adhesive layer 16 of the first example.

As shown in (b), the operation member 53 may rotate by an external force until it coincides with the opening 54a of the housing 54. When the operation member 53 coincides with the opening 54a of the housing 54, the operation member 53 slides along the opening 54a of the housing 54 so as to prevent rotation or horizontal distortion when the microneedle array 55 uniformly vertically descends.

In this case, the skin in contact with the side wall of the housing 54 may be expanded to the outside by the supporting force of the operation member 53. As a result, by inserting the microneedle into the expanded skin, it is possible to improve the insertion efficiency and ensure the insertion safety at the same time.

Subsequently, in the operation member 53, the microneedle array 55 protrudes to the outside of the housing 54. Accordingly, the microneedle array 55 is in contact with the skin, and the microneedle is inserted into the skin by a force of releasing the supporting force of the operation member 53.

As such, the microneedle applicator 50 according to the fifth example of the present invention may provide an external force to the microneedle array 55 through the release of the supporting force by the edge 53a of the operation member 53. Therefore, since the microneedle applicator 50 according to the fifth example of the present invention may provide an external force with a relatively simple structure, it is possible to reduce the manufacturing cost, and thus improve the price competitiveness.

Although an exemplary embodiment of the present invention has been described above, the spirit of the present invention is not limited to the exemplary embodiment presented in the present specification, and those skilled in the art who understand the spirit of the present invention will be able to easily suggest other exemplary embodiments by modifying, changing, deleting or adding components within the scope of the same spirit, but this is also said to be within the scope of the present invention.

## Claims

1. A microneedle applicator, comprising:
a microneedle array;
an operation member disposed on the upper portion of the microneedle array and operating such that a user applies an external force so as to insert a microneedle into skin;
a housing for accommodating the microneedle array and the operation member;
a first guide member disposed in at least one position on a lateral portion of the operation member or on a lateral portion of an interworking member of the operation member; and
a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

2. The microneedle applicator of claim 1, wherein the microneedle has a hardness of 0.5N or less.

3. The microneedle applicator of claim 2, wherein the microneedle array comprises a drug, and
wherein the drug comprises a drug for local anesthesia selected from the group consisting of cocaine, procaine, chloroprocaine, tetracaine, dibucaine, lidocaine, mepivacaine, benzocaine, bupivacaine and a combination thereof.

4. The microneedle applicator of claim 1, wherein the first guide member is provided in a straight-line shape in a vertical direction from a lateral portion of the operation member.

5. The microneedle applicator of claim 4, wherein the second guide member has a different width depending on a position on the horizontal cross-section of the housing.

6. The microneedle applicator of claim 1, further comprising an adhesive layer provided on the lower portion of the housing and attached to the skin,
wherein the adhesive layer has an opening in the center and is provided with a peeling part such that one side protrudes to the outside.

7. The microneedle applicator of claim 1, further comprising a locking member provided to support the lower end of the operation member,
wherein the operation member presses the microneedle array with a force of passing through the locking member by the external force.

8. The microneedle applicator of claim 7, wherein the locking member is provided to protrude from the upper end of the inner surface of the housing toward the center.

9. The microneedle applicator of claim 7, wherein the locking member is vertically provided in a rod shape from the inside of the housing,
wherein the operation member is provided with a groove portion at a position corresponding to the locking member, and is provided with a support to protrude into the groove portion, and
wherein the support is broken by the external force.

10. The microneedle applicator of claim 7, wherein the locking member is a groove portion provided on the upper end of the inner surface of the housing, and
wherein the operation member is provided with a protrusion that is inserted into the groove portion along the outer periphery.

11. The microneedle applicator of claim 1, further comprising a fluid layer provided between the operation member and the microneedle array,
wherein the fluid layer supports the lower end of the operation member, and
wherein the operation member presses the microneedle array with a force of destroying the fluid layer by the external force.

12. The microneedle applicator of claim 11, wherein the fluid layer comprises air, a liquid and a drug.

13. The microneedle applicator of claim 1, further comprising a rubber band provided between the operation member and the microneedle array,
wherein the operation member fixes the rubber band such that the rubber band has elasticity on the upper side of the microneedle array, and
wherein when the rubber band is released by the external force, the operation member presses the microneedle array by an elastic restoring force of the rubber band.

14. The microneedle applicator of claim 1, further comprising a leaf spring provided between the operation member and the microneedle array,
wherein the operation member presses the microneedle array with a force of elastically deforming the leaf spring by the external force.

15. The microneedle applicator of claim 14, further comprising a support for supporting the microneedle array between the leaf spring and the microneedle array

16. The microneedle applicator of claim 1, wherein the microneedle array is a microneedle patch.

17. A microneedle applicator, comprising:
a microneedle array;
an operation member disposed on the upper portion of the microneedle array and operating such that a user applies an external force so as to insert a microneedle into skin;
a housing for accommodating the microneedle array and the operation member; and
a support member provided between the microneedle array and the operation member to support the operation member with a constant supporting force and release the supporting force by the external force such that the operation member joins the microneedle array.

18. The microneedle applicator of claim 17, further comprising:
a first guide member disposed in at least one position on a lateral portion of the operation member; and
a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

19. The microneedle applicator of claim 17, wherein the support member is a locking member provided to protrude from the upper end of the inner surface of the housing toward the center,
wherein the locking member supports the lower end of the operation member, and
wherein the operation member presses the microneedle array with a force of releasing a supporting force by the locking member by the external force.

20. The microneedle applicator of claim 17, wherein the support member is a fluid layer, and
wherein the operation member presses the microneedle array with a force of releasing a supporting force by the fluid layer by the external force.

21. The microneedle applicator of claim 17, wherein the skin in contact with the housing expands to the outside by the support force.

22. A microneedle applicator, comprising:
a microneedle array;
an operation member disposed on the upper portion of the microneedle array and operating such that a user applies an external force so as to insert a microneedle into skin;
a housing for accommodating the microneedle array and the operation member; and
an elastic member provided between the microneedle array and the operation member to press the microneedle array with an elastic force caused by the external force.

23. The microneedle applicator of claim 22, further comprising:
a first guide member disposed in at least one position on a lateral portion of the operation member or on a lateral portion of an interworking member of the operation member; and
a second guide member which is disposed on the inner surface of the housing in a position corresponding to the first guide member so as to be engaged with the first guide member, and allows the first guide member to slide so as to prevent the microneedle array from rotating or horizontally distorting when the microneedle array uniformly vertically descends due to the external force.

24. The microneedle applicator of claim 22, wherein the elastic member is a rubber band,
wherein the operation member fixes the rubber band such that the rubber band has elasticity on the upper side of the microneedle array, and
wherein when the rubber band is released by the external force, the operation member presses the microneedle array by an elastic restoring force of the rubber band.

25. The microneedle applicator of claim 22, wherein the elastic member is a leaf spring, and
wherein when the leaf spring is pressed by the external force, the operation member presses the microneedle array by an elastic deformation force of the leaf spring.

26. A microneedle array applicator, comprising:
a microneedle array;
an operation member disposed on the upper portion of the microneedle array and operating such that a user applies an external force so as to insert a microneedle into skin; and
a housing for accommodating the microneedle array and the operation member,
wherein the operation member and the opening of the housing have the same shape, and the operation member is disposed to be staggered at a predetermined angle such that the operation member is supported by an opening edge of the housing, and
wherein the support of the operation member is released according to the rotation of the operation member by the external force such that the operation member slides along the opening of the housing so as to prevent the microneedle array from rotating when the microneedle array uniformly vertically descends.
